# EUROPEAN PATENT SPECIFICATION

(11) **EP 2 693 213 B1**
(45) Date of publication and mention of the grant of the patent: **26.07.2017**
(21) Application number: 12764272.6
(22) Date of filing: 29.03.2012
(51) Int. Cl.: G01N 33/543, G01N 33/558

(54) **DETECTION METHOD USING IMMUNOCHROMATOGRAPHY CAPABLE OF DETERMINING SAMPLE WITHOUT ADDITION OF SPECIMEN AS OPERATION-FAILURE SAMPLE, AND TEST STRIP FOR USE IN SAME**
ERKENNUNGSVERFAHREN DURCH IMMUNCHROMATOGRAPHIE ZUR BESTIMMUNG EINER PROBE ALS FEHLPROBE OHNE ZUSATZ EINES PRÜFMITTELS UND TESTSTREIFEN ZUR VERWENDUNG IN DIESEM VERFAHREN
MÉTHODE DE DÉTECTION FAISANT APPEL À L'IMMUNOCHROMATOGRAPHIE ET PERMETTANT D'IDENTIFIER UN ÉCHANTILLON TOUT EN DÉTECTANT TOUT DÉFAUT D'ADDITION D'UN SPÉCIMEN, ET BANDELETTE D'ESSAI UTILISABLE À CET EFFET

(30) Priority: 31.03.2011 JP 2011077149
(43) Date of publication of application: 05.02.2014
(73) Proprietor: Sekisui Medical Co., Ltd., Tokyo 103-0027 (JP)
(72) Inventor: MORITA, Motoki, Ryugasaki-shi Ibaraki 301-0852 (JP); KOBAYASHI, Koji, Ryugasaki-shi Ibaraki 301-0852 (JP); ITO, Sachiko, Ryugasaki-shi Ibaraki 301-0852 (JP)
(74) Representative: Hoffmann Eitle
(86) International application number: PCT/JP2012/058302
(87) International publication number: WO 2012/133615

(56) References cited:
- EP-A1- 0 512 390
- EP-A1- 1 347 299
- EP-A1- 2 554 990
- WO-A1-2006/041537
- WO-A1-2010/001598
- JP-A- 2000 055 918

## Description

### TECHNICAL FIELD

The present invention relates to an immunochromatographic method of detecting an analyte in a sample acquired by diluting a specimen. In particular, the present invention relates to an immunochromatographic detection method capable of recognizing the failure to add a specimen by detecting the presence or absence of a component that is contained in the specimen and that is different from the analyte and a test strip and a test kit used therewith.

### BACKGROUND ART

Clinics and small hospitals recently have increasing needs for "conducting various tests during patient examination" and tests are increasingly conducted as point-of-care testing (POCT) instead of conventional outsourced examination. Representative examples of POCT reagents include a lateral flow immunochromatographic test strip (Patent Document 1). A typical immunochromatographic test strip has a test line for detecting an analyte and a control line indicative of achievement of reaction (equivalent to the second control line in the present invention described later; the same applies to the following description of "BACKGROUND ART"). The complex of an analyte and a label to which an antibody to the analyte is immobilized will be captured by an antibody immobilized onto an insoluble membrane to form the test line while the remaining label not captured at the test line will be captured by anti-immunoglobulin antibodies at the control line to form the control line.

Measuring methods using an immunochromatographic detection method include a qualitative method in which the coloring of test line is visually determined and a quantitative method in which the coloring intensity of test line is measured by a dedicated device, and the immunochromatographic test strip and device corresponding to respective methods are used in examinations in clinics and small hospitals.

For the quantitative method in which the coloring intensity of test line of immunochromatographic test strip is measured by a dedicated device, various pretreatments may be performed depending on the type of specimen, the amount of specimen used, and the measurement principle of the reagent. Therefore, application of a sample to the test strip or device may be performed, for example, by directly dripping a specimen, by dripping a specimen diluted with a dedicated diluting solution, or by dripping a small amount of specimen before dripping a dedicated diluting solution. Among these cases, the dilution of specimen by a dedicated diluting solution is performed, for example, when it is difficult to obtain a large amount of specimen as in the case of specimen from infants or children or when an analyte in specimen is at high concentration and the concentration of the analyte is adjusted to a concentration suitable for detection.

If a blood specimen including red blood cells is diluted, the coloring due to hemoglobin occurs in a sample acquired by diluting the specimen and, therefore, whether the sample includes the specimen can visually be confirmed. However, if a relatively light-colored specimen such as plasma, serum, and urine is diluted at a high dilution factor, it is difficult to distinguish a diluted sample solution from a specimen-dilution solution itself with the naked eye. Therefore, the specimen may be forgotten to be added and only the specimen-dilution solution may be dripped as a sample onto a test strip.

However, since the control line of a typical immunochromatographic test strip is formed by capturing the label not used in reaction with the test line, the control line emerges even if only the dedicated diluting solution is dripped onto the test strip. Therefore, in the case of a sample consisting only of the diluting solution because of the failure to add a specimen, the test is considered accomplished, resulting in a negative result or the concentration determined as "zero". Thus, this causes an incorrect diagnosis.

As described above, an immunochromatographic detection method comprising a step of detecting the failure to add a specimen and an immunochromatographic test strip comprising a means for detecting the failure to add a specimen has never been reported.

### CITATION LIST

### PATENT LITERATURE

Patent Literature 1: Japanese Unexamined Patent Application Publication (Translation of PCT Application) No. 2007-524813

### SUMMARY OF INVENTION

### TECHNICAL PROBLEM

An object of the present invention is to provide an immunochromatographic detection method comprising a step of detecting the failure to add a specimen and an immunochromatographic test strip comprising a means for detecting the failure to add a specimen. It is therefore an object of the present invention to provide an immunochromatographic detection method of detecting an analyte in a sample acquired by diluting a light-colored specimen such as plasma, serum, and urine in which if a sample without addition of specimen is measured, the sample is determined as an improperly operated sample so as to enable detection of the failure to add a specimen to a diluting solution, and an immunochromatographic test strip used therewith.

### SOLUTION TO PROBLEM

The inventers have found that, in an immunochromatographic detection method of detecting an analyte in a sample acquired by diluting a specimen, it is possible to detect the failure to add the specimen by capturing, with an antibody solid-phased on an insoluble membrane to, the complex of a component contained in the specimen other than the analyte (control component) and a label to which an antibody to the component is immobilized and detecting the presence or absence of the control component, thereby completing the present invention.

Therefore, the present invention is configured as follows.
[1] An immunochromatographic detection method of detecting an analyte in a sample acquired by diluting a specimen, comprising the steps of:
   1) providing the sample to a sample pad of an immunochromatographic test strip comprising
      a) a sample pad,
      b) a conjugate pad disposed downstream relative to the sample pad, the conjugate pad comprising
         a label to which a first antibody to an analyte is immobilized, and
         a label to which a first antibody to a control component is immobilized, the control component being contained in the specimen and different from the analyte, and
      c) an insoluble membrane which is disposed downstream relative to the conjugate pad and to which a second antibody to the analyte and a second antibody to the control component are immobilized;
   2) detecting on the insoluble membrane the presence or absence of the complex of the analyte and the first and second antibodies to the analyte;
   3) detecting on the insoluble membrane the presence or absence of the complex of the control component and the first and second antibodies to the control component; and
   4) determining that no specimen is contained in the sample if no complex can be detected at the detecting step of 3),
      wherein the specimen is plasma or serum, and wherein the control component is hemoglobin.
[2] The detection method of [1] above, wherein the item (b) of the immunochromatographic test strip further comprise a second control component not contained in the sample, wherein the item (c) of the immunochromatographic test strip further comprises a detection reagent for the second control component, and wherein the method further comprises the step of
   5) detecting on the insoluble membrane the presence or absence of the second control component.

### ADVANTAGEOUS EFFECTS OF INVENTION

The present invention provides an immunochromatographic detection method of detecting an analyte in a sample acquired by diluting plasma, serum, urine, etc., and a test strip used therewith. The method and the test strip are capable of detecting a sample in which a specimen is forgotten to be added to a diluting solution and determining such a sample as an improperly operated sample. Therefore, incorrect determination can be reduced by determining false negatives and false low values (concentrations less than detection limit) that are caused because a medical worker unfamiliar with examination forgets to add a specimen, as improperly operated samples.

### BRIEF DESCRIPTION OF DRAWINGS

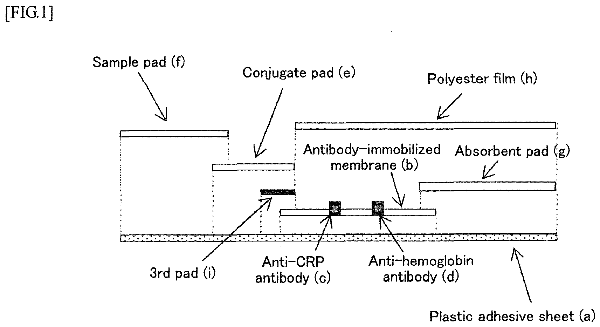

[Fig. 1] Fig. 1 is a schematic of the structure of a test strip for an example of the present invention.

### DESCRIPTION OF EMBODIMENTS

### (Detection method)

An immunochromatographic detection method of the present invention is a method of detecting an analyte in a sample acquired by diluting a specimen and comprising the following steps 1) to 4):
1) a step of providing the sample to a sample pad of an immunochromatographic test strip comprising the following a), b), and c), i.e.,
   a) a sample pad,
   b) a conjugate pad disposed downstream relative to the sample pad, the conjugate pad comprising
      a label to which a first antibody to an analyte is immobilized, and
      a label to which a first antibody to a control component is immobilized, the control component being contained in specimen and different from the analyte, and
   c) an insoluble membrane which is disposed downstream relative to the conjugate pad and to which a second antibody to the analyte and a second antibody to the control component are immobilized;
2) a step of detecting on the insoluble membrane the presence or absence of the complex of the analyte and the first and second antibodies to the analyte;
3) a step of detecting on the insoluble membrane the presence or absence of the complex of the control component and the first and second antibodies to the control component; and
4) a step of determining that no specimen is contained in the sample if no complex can be detected at the detecting step of 3).

The step of 1) is a step using an immunochromatographic test strip. Although a typical test strip includes only a detection reagent for detecting an analyte, the present invention is characterized in that also included is a detection reagent for detecting a component that is contained in the specimen and different from the analyte (control component).

By using such a test strip comprising a means for detecting the control component, whether a specimen is contained in the sample can be determined through step 2) of detecting the analyte in the sample and step 3) of detecting the control component in the sample. Therefore, if the control component is not detected at step 3), it can be determined at step 4) that no specimen is contained in the sample. If the control component is detected, even when the analyte is not detected at step 2), it can be determined that the specimen is contained in the sample. To enable detection of the presence or absence of provision of a sample itself, a conventional means for detecting a control component can naturally be combined with this test strip (a second control component described later).

In the immunochromatographic detection method of the present invention, the complex of an analyte and a label to which an antibody to the analyte is immobilized is captured by an antibody to the analyte immobilized onto the insoluble membrane of the test strip, and thus forms the test line. The complex of a control component and a label to which an antibody to the control component is immobilized is captured by an antibody to the control component immobilized onto the same insoluble membrane and forms the control line. If neither the test line nor the control line is formed, it can be determined that the specimen itself is not contained in the sample, i.e., the sample is a sample without addition of the specimen (improperly operated sample) rather than that the analyte is not contained in the specimen. If both the test line and the control line are formed, it can be determined that the analyte is contained in the specimen. If the test line is not formed while the control line is formed, it can be determined that the analyte is not contained in the specimen. Therefore, if the analyte is not detected at step 2), a determination can be made by judging whether the analyte is indeed not contained in the specimen (i.e., a negative result is indicated) or the specimen is forgotten to be added to the diluting solution.

The present invention can provide a method and a reagent capable of detecting whether the specimen is forgotten to be added to the diluting solution depending on the presence or absence of detection of a control component and determining the sample to which the specimen is forgotten to be added as an improperly operated sample.

### (Analyte)

The analyte of the present invention is a substance present in blood, plasma, serum, spinal fluid, and urine and is exemplarily illustrated as, for example, C-reactive protein (CRP), inflammation related markers such as IgA, IgG, and IgM, coagulation/fibrinolysis markers such as fibrin degradation products (e.g., D-dimer), soluble fibrin, thrombin/anti-thrombin complex (TAT), and plasmin/plasmin-inhibitor complex (PIC), circulation related markers such as oxidized LDL and brain natriuretic peptide (BNP), metabolism related markers such as adiponectin, tumor markers such as carcinoembryonic antigen (CEA), α-fetoprotein (AFP), CA19-9, CA125, and prostate-specific antigen (PSA), infection related markers such as hepatitis B virus (HBV), hepatitis C virus (HCV), Chlamydia trachomatis, and gonococcus, allergen-specific immunoglobulin E (IgE), hormones, and drugs.

### (Control Component)

The control component of the present invention may be any component contained in the specimen and different from the analyte, and is desirably a component invariably contained in the specimen. Specifically, the control component may be a protein in blood such as albumin, globulin, and hemoglobin and a protein in urine such as urinary albumin.

The inventers have found that since a slight amount of hemoglobin is contained in plasma and serum specimens, if a plasma or serum specimen is diluted for measurement, hemoglobin can be utilized as the control component to form the control line.

The immunochromatographic test strip of the present invention enables detection at a concentration equal to or greater than 10 pg/mL, though depending on the property of the analyte and the antibody to the analyte. Therefore, any substance invariably present in blood at 1 µg/mL or higher regardless of the presence or absence of disease can be detected as an analyte captured by a test line and can be detected as a control component captured by a control line.

### (Specimen and Sample)

The specimen of the present invention refers to biological fluid such as blood, plasma, serum, spinal fluid, and urine, and a specimen diluted with a specimen-dilution solution is referred to as a sample.

The dilution of a specimen will be described in detail by taking as an example the case that the analyte is C-reactive protein (CRP) while the control component is hemoglobin (Hb). The dilution of a specimen is performed in consideration of concentration suitable for measurement of the analyte, and a dilution factor is generally 2 to 10,000.

With regard to a test strip for CRP measurement described later as an example of the present invention, infants and children are defined as measurement subjects and the amount of specimen is designed to be 1 to 10 µL. To ensure the amount of sample necessary for detection through immunochromatography, the specimen must therefore be diluted by a factor of 20 to 200 with a specimen-dilution solution.

The immunochromatographic test strip and test kit of the present invention will be described in detail.

### (Antibody to Analyte Used in the Present Invention)

The antibody to an analyte used in the present invention may be any antibody specifically reactive to the analyte, is not limited in any way by a method of producing the antibody, and may be a polyclonal antibody or a monoclonal antibody. For example, if the analyte is human CRP, an anti-human CRP antibody may be any antibody specifically reactive to human CRP, is not limited in any way by a method of producing the antibody, and may be a polyclonal antibody or a monoclonal antibody. A hybridoma producing the antibody can generally be prepared by the cell fusion between spleen cells of an animal immunized by human CRP and myeloma cells from the same species in accordance with a method of Kohler and Milstein (see Nature, Vol. 256, p.495, 1975).

If the antibody used in the present invention is a monoclonal antibody, the relationship between an antibody to be immobilized to a label (first antibody) and an antibody to be immobilized to an insoluble membrane (second antibody) is as follows. If the epitope of the first antibody is monovalent, the epitope of the second antibody shall be different from the first antibody, and if the epitope of the first antibody is multivalent, the epitope of the second antibody may be the same as the first antibody, or the first antibody may be the same antibody as the second antibody.

When a specimen diluted by a factor of 20 to 200 as described above is used as a sample, a combination of antibodies are desirably used such that the measurement at the CRP concentration of 0.2 to 20 mg/dL can be performed at a dilution factor of about 100. For example, a monoclonal antibody produced by the hybridoma of accession number FERM BP-11344 and a monoclonal antibody produced by the hybridoma of accession number FERM BP-11345 may be used as a combination of CRP measurement antibodies: the former as the antibody to be immobilized to a label and the latter as the antibody to be immobilized to an insoluble membrane.

### (Antibody to Control Component)

The antibody to a control component used in the present invention may be any antibody specifically reactive to the control component, is not limited in any way by a method of producing the antibody, and may be a polyclonal antibody or a monoclonal antibody. For example, if the control component is human Hb, an anti-human Hb antibody may be any antibody specifically reactive to human Hb, is not limited in any way by a method of producing the antibody, and may be a polyclonal antibody or a control component antibody. The other details such as a manufacturing method of a monoclonal antibody, a condition related to combination of antibodies, etc., conform to the description related to the anti-CRP antibody.

A combination of the anti-human Hb antibodies may be a combination of anti-human Hb monoclonal antibodies acquired from two hybridomas #69202 and #69209 produced by immunizing mice with human Hb by the present inventors in the usual manner, for example, or may be an appropriate combination selected as needed from commercially available anti-human Hb antibodies.

### (Sample Pad)

In the present invention, a "sample pad" is a part receiving a sample, shaped into a pad to absorb a liquid sample, and comprises any material and form as long as it allows the passage of liquid and the analyte. Specific examples of materials suitable for sample pad include, but not limited to, glass fibers, acrylic fibers, hydrophilic polyethylene materials, dry papers, paper pulp, and fabrics. A glass fiber pad is preferably used. The sample pad may additionally be given a function of a conjugate pad described later. For the purpose of prevention or suppression of non-specific reaction (adsorption) in an antibody-immobilized membrane, the sample pad may contain a commonly used blocking reagent.

For the blocking reagent, a reagent having no adverse effect on the specific reaction itself can appropriately be selected from, for example, NEO PROTEIN SAVER (manufactured by TOYOBO), sericin, ImmunoBlock^{™} (manufactured by Dainippon Pharmaceutical), Applie Block (manufactured by Seikagaku Biobusiness Corporation), SEA BLOCK^{™}/EIA/WB (manufactured by PIERCE), Blocking One (manufactured by NACALAI TESQUE), BSA, Blocking Peptide Fragment (manufactured by TOYOBO), Starting Block^{™} (PBS) Blocking Buffer (manufactured by PIERCE), Smart Block^{™} (manufactured by CANDOR bioscience GmbH), and HeteroBlock (manufactured by Omega Biologicals).

### (Label)

Known materials normally known as carriers for immobilizing antibody in an immunochromatographic assay can be used for the label. For example, colloidal gold particles, colloidal platinum particles, color latex particles, and magnetic particles are preferable and the colloidal gold particles are particularly preferable.

The particle diameter of colloidal gold particles is known to significantly affect the sensitivity of measurement based on immunochromatography and the particle diameter of colloidal gold particles of the present invention is preferably 20 to 60 nm and particularly preferably 30 to 40 nm. The colloidal gold can be manufactured with a generally known method, for example, by dripping and stirring a trisodium citrate aqueous solution in a heated tetrachloroauric(III) acid aqueous solution.

The case of using the colloidal gold particles will hereinafter be described in detail.

### (Sensitization of Antibody to Label)

The immobilization of antibody against analyte or control component to colloidal gold, for example, the immobilization of an antibody to CRP or Hb to colloidal gold is normally achieved by physisorption. In this case, the antibody concentration is preferably prepared to 0.5 µg/mL to 5 µg/mL and the buffer solution and pH are preferably a 2 mmol/L phosphate buffer solution (pH 6 to 7) or a 2 mmol/L borate buffer solution (pH 8 to 9) and more preferably a 2 mmol/L phosphate buffer solution (pH 7.0). The region on the colloidal gold without bound antibody is preferably blocked by binding with BSA etc. The colloidal gold-labeled antibody produced in this way is dispersed and preserved in a preservation reagent containing a component for inhibiting denaturalization (denaturalization inhibiting agent). Proteins such as BSA, glycerin, sugar, etc., are used for this denaturalization inhibiting agent.

In this description, a "conjugate" refers to a label to which an antibody is immobilized such as an anti-CRP monoclonal antibody that is the antibody to the analyte as described above, an anti-Hb monoclonal antibody that is the antibody to the control component, etc.

### (Detection Reagent)

In the present invention, specifically, a "detection reagent" is a solution containing at least a conjugate.

A detection reagent may contain, for example, one or more stabilizers, solubilizers, etc., for the purpose of maintaining the conjugate in a stable state so as to facilitate the specific reaction between the antibody immobilized to the conjugate and the analyte (e.g., CRP) or the control component (e.g., Hb) or to make the conjugate dissolved and fluidized promptly and effectively when mixed with the sample. The stabilizers, solubilizers, etc., can include bovine serum albumin (BSA), sucrose, casein, and amino acids, for example.

The term "detection" or "measurement" as used herein must be construed in the broadest sense including verification of the presence and/or quantification of an analyte, for example, CRP, and a control component, for example, Hb, and must not be construed in a limited manner in any sense.

### (Conjugate Pad)

In the present invention, a "conjugate pad" refers to a pad acquired by drying a material suitable for conjugate pad described later after impregnating the material with a detection reagent specifically reactive with an analyte, for example, a detection reagent specifically reactive with CRP, and/or a detection reagent specifically reactive with a control component, for example, a detection reagent specifically reactive with Hb. The conjugate pad has a function of allowing the detection reagent and CRP or Hb to form a complex when the sample passes through the conjugate pad. The conjugate pad may be disposed in contact with an anti-CRP antibody- and anti-Hb antibody-immobilized membrane by itself. Alternatively, the conjugate pad may be disposed in contact with a sample pad so as to receive the sample passing through the sample pad as a capillary flow and then transfer the sample as a capillary flow to another pad (hereinafter referred to as a "3rd pad") in contact with a surface different from the contact surface for the sample pad. The selection of one or more parts of the sample pad and the conjugate pad and how the selected parts are disposed on the antibody-immobilized membrane may be changed as appropriate.

Materials suitable for the conjugate pad include, but not limited to, paper, a cellulose mixture, nitrocellulose, polyester, an acrylonitrile copolymer, glass fibers, and nonwoven fibers such as rayon. A glass fiber pad is preferably used.

The conjugate pad may contain, for example, one or more stabilizers, solubilizers, etc., for the purpose of maintaining the detection reagent in a stable state and facilitating the specific reaction of the detection reagent and the analyte (e.g., CRP) or the control component (e.g., Hb) in the sample or achieving prompt and effective dissolution and fluidization when the detection reagent contacts the sample. The stabilizers, solubilizers, etc., can include bovine serum albumin (BSA), sucrose, casein, and amino acids, for example. In particular, an anti-CRP antibody might have different reactivity between in the presence and absence of Ca²⁺ ions and the conjugate pad may contain a chelate agent of Ca²⁺ ions such as EDTA and EGTA as appropriate so as to control the reactivity or, conversely, calcium salts such as CaCl₂ may be added in order to add Ca²⁺ ions.

### (3rd Pad)

In the present invention, a 3rd pad can be disposed for the purpose of removing components unnecessary for detection of an analyte (e.g., CRP) and a control component (e.g., Hb) out of reaction components of the sample and the detection reagent so that components necessary for reaction can smoothly develop/spread in the antibody-immobilized membrane. For example, blood cells, insoluble blood cell debris, etc., are desirably removed as components unnecessary for the detection of CRP or Hb. The 3rd pad may also be given an additional effect of preliminarily removing, among agglutinations generated by an antigen-antibody reaction, agglutinations growing to a size preventing the movement to and the smooth development/spread in the antibody-immobilized membrane. The 3rd pad includes any material or form allowing the passage of liquid and sample components. Specific examples are, but not limited to, glass fibers, acrylic fibers, hydrophilic polyethylene materials, dry papers, paper pulp, fabrics, etc. A blood cell separation membrane or a similar membrane is preferably used.

### (Immobilization of Antibody to Insoluble Membrane)

In the immunochromatographic test strip of the present invention, the antibody to an analyte (e.g., CRP) or a control component (e.g., Hb) can be immobilized to an insoluble membrane with a commonly known method. For example, in the case of a flow-through format, the antibody is prepared at a predetermined concentration and a certain amount of the solution thereof is applied to the insoluble membrane at a point or in the shape of a certain symbol such as "+". In the case of a lateral flow format, the antibody is prepared at a predetermined concentration and the solution thereof is applied to the insoluble membrane in a line shape by using a device having a mechanism capable of horizontally moving while discharging the solution from a nozzle at a constant rate.

In this case, the concentration of antibody is preferably 0.1 mg/mL to 5 mg/mL and more preferably 0.5 mg/mL to 2 mg/mL. The amount of immobilized antibody on the insoluble membrane can be optimized by adjusting an application amount dripped onto the insoluble membrane in the case of a flow-through format, and can be optimized by adjusting a discharge rate from the nozzle of the device in the case of a lateral flow format. Particularly, in the case of a lateral flow format, 0.5 µL/cm to 2 µL/cm is preferable. In the present invention, a "flow-through membrane assay" refers to a format in which the sample liquid etc., spread so as to perpendicularly pass through the insoluble membrane and a "lateral flow membrane assay" refers to a format in which the specimen liquid etc., spread so as to move in parallel with the insoluble membrane.

In the present invention, the positions of application of antibodies to an analyte (e.g., CRP) or a control component (e.g., Hb) to the insoluble membrane may be placed such that the detection reagent spreads from the conjugate pad by capillarity and sequentially passes through the lines to which the respective antibodies are applied in the case of a lateral flow format. Preferably, the line with an anti-CRP antibody applied is located upstream while the line with an anti-Hb antibody applied is located downstream thereof. In this case, it is desirable to place a sufficient distance between the respective lines such that signals of labels can be detected. In the case of a flow-through format, the positions of application of antibodies to CRP or Hb may be placed such that signals of labels can be detected.

An antibody solution applied to the insoluble membrane can normally be prepared by using a predetermined buffer solution. The types of buffer solution include commonly used buffer solutions such as phosphate buffer solution, Tris buffer solution, and Good's buffer solution. The buffer solution preferably has pH in a range of 6.0 to 9.5, more preferably 6.5 to 8.5, further preferably 7.0 to 8.0. The buffer solution may contain salts such as NaCl, stabilizer and preservative such as sucrose, and antiseptic such as ProClin. The salts include those contained for adjusting ionic strength, such as NaCl, as well as those added at a step of adjusting pH of the buffer solution, such as sodium hydroxide.

After antibody(ies) is immobilized to the insoluble membrane, the blocking can be performed by using a commonly used blocking agent in a solution or mist form to cover the portion to which antibody is not immobilized. In this description, an insoluble membrane to which an antibody is immobilized as described above is also referred to as an "antibody-immobilized membrane".

### (Insoluble Membrane)

In the present invention, the insoluble membrane (hereinafter also simply referred to as the membrane) may be of any material. For example, the materials include, but not limited to, polyethylene, polyethylene terephthalate, nylons, glass, polysaccharide such as cellulose and cellulose derivatives, or ceramics. Specific examples include glass fiber filter paper and cellulose filter paper available from Millipore Corporation, Toyo Roshi Kaisha, Ltd., and Whatman^{™}. The speed of flow through the membrane of the immune complex of a colloidal gold-labeled antibody and an analyte (e.g., CRP) can be controlled by appropriate selections of the pore diameter, structure, etc., of the insoluble membrane. Since the amount of labeled antibody held by an antibody immobilized to the membrane can be adjusted by controlling the speed of flow through the membrane, the pore diameter and the structure of the membrane are desirably optimized by considering the compatibility with the other constituent materials of the immunochromatographic test strip of the present invention.

### (Absorbent pad)

In the present invention, an "absorbent pad" refers to a liquid-absorbing part absorbing the sample migrated or passed through the insoluble membrane to control the spread of the sample. In a lateral flow format, the absorbent pad may be disposed at the most downstream portion of the test strip configuration, and in a flow-through format, the absorbent pad may be disposed on the lower portion of the antibody-immobilized membrane, for example. For example, the absorbent pad may be, but are not limited to, filter paper. Preferably, 740-E of Whatman^{™} etc., are used.

### (Test Strip)

In the present invention, a "test strip" may be any strip including at least an insoluble membrane to which an antibody to an analyte is immobilized and further containing a reagent component and other membranes etc., as needed. Other membranes may be a sample pad, a conjugate pad, an absorbent pad, etc. The test strip is usually arranged on a solid phase support such as a plastic adhesive sheet. It is obvious that the solid phase support is made of material not hindering the capillary flow of the sample and that the adhesive component is made of material not hindering the capillary flow of the sample. A polyester film etc., can be used for lamination in order to increase the mechanical strength of the antibody-immobilized membrane and to prevent evaporation of water (drying) during the assay.

The test strip may be used after stored in or mounted on an appropriate container (housing) with respect to the size of the strip, the manner and position of the addition of the sample, the position of immobilization of antibody on the antibody-immobilized membrane, the method of signal detection, etc., and such a stored or mounted state is referred to as a "device".

### (One and the Same Test Strip)

The strip for detecting an analyte may be one and the same strip as, or a separate strip different from, the strip for detecting a control component. Therefore, in the case of one and the same strip, the strip is made up of one and the same conjugate pad containing a label to which a first antibody to a control component is immobilized and a label to which a first antibody to an analyte is immobilized, and one and the same insoluble membrane to which a second antibody to the control component and a second antibody to the analyte are immobilized, as described above. If different separate strips are used, the same sample is measured by using a strip for measuring an analyte made up of a conjugate pad containing a label to which a first antibody to an analyte is immobilized and an insoluble membrane to which a second antibody to the analyte is immobilized, and a strip for detecting a control component made up of a conjugate pad containing a label to which a first antibody to a control component is immobilized and an insoluble membrane to which a second antibody to the control component is immobilized. When one and the same strip is used, the size can be reduced and the measurement can easily be performed. On the other hand, if separate strips are used, a plurality of strips for different analytes can be combined as needed and, therefore, the versatility of individual strips is thought to be improved. Even when strips are separated, the strips can obviously be housed in the same housing to form one device.

In this description, the "insoluble membrane" is also referred to as a "solid phase" and, allowing, or a state of allowing, the insoluble membrane to physically or chemically supporting an antigen or an antibody may be expressed as "immobilization", "immobilized", "solid-phased", "sensitization", or "absorption".

### (Specimen-dilution solution)

A diluting solution of any composition may be used in the present invention as long as the diluting solution does not significantly inhibit the antigen-antibody reaction of analyte or control component with respective antibodies or, conversely, does not significantly facilitate the reaction resulting in excessive agglutinations of the lables which deteriorates the spread by capillarity, and enables the detection of the antigen-antibody reaction signal depending on the concentration of antigen. Such a diluting solution may be purified water or a low-concentration buffer solution at pH 6.0 to 10.0, for example. The low-concentration buffer solution may be, for example, a 10 to 20 mmol/L phosphate buffer solution, a 10 to 20 mmol/L Tris-HCl buffer solution, and a 10 to 20 mmol/L glycine-HCl buffer solution.

A surfactant can be added to these diluting solutions in order to control the spread rate of the sample liquid in the strip. Particularly, in the system of measuring CRP as an example of the analyte, if the monoclonal antibody produced by the hybridoma of the accession number FERM BP-11344 is used as a label-immobilized antibody and the monoclonal antibody produced by the hybridoma of the accession number FERM BP-11345 is used as an insoluble membrane-immobilized antibody, the diluting solution can contain sodium alkylsulfate expressed by a general formula CH₃(CH₂)ₙOSO₃Na (n=5 to 10) to adjust the range of measurement. Preferably, it is desirable to add 0.05 to 0.3 % sodium hexylsulfate, sodium octylsulfate, etc., since a preferable concentration-reaction curve is acquired. In this case, a desirable dilution factor is 50 to 200. The diluting solution may further contain a chelate agent of Ca²⁺ ions such as EDTA and EGTA.

### (Second Control Component)

In the immunochromatographic detection method of the present invention, a so-called conventional control component (referred to as a second control component in the present invention) can obviously be used. Therefore, the configuration of an immunochromatographic test strip can be implemented by employing a configuration with a conjugate pad further containing a second control component that is a component not contained in the sample and an insoluble membrane further containing a reagent for detecting the second control component.

The second control component contained in the conjugate pad may be, for example, an antibody labeled with a label and not reactive with the analyte, and a highly antigenic protein such as KLH (keyhole limpet hemocyanin) labeled with a label. These second control components are components (substances) having no possibility of being present in the sample and can appropriately be selected to suitably correspond to an antibody to a control component (a control component capture antibody).

With regard to a reagent immobilized to the insoluble membrane for detecting the second control component, for example, if labeled KLH is contained as the second control component in the conjugate pad, an anti-KLH antibody etc., correspond to the detection reagent for the second control component. Although the position of immobilization of the detection reagent to the membrane can appropriately be selected, the detection reagent is preferably disposed downstream relative to the detection reagent of the analyte.

With this configuration, if the second control component is not detected, it can also be determined that no sample is provided to the test strip.

### EXAMPLES

The present invention will specifically be described by giving an example of detecting CRP as the analyte and Hb as the control component; however, the scope of the present invention is not limited to the example.

### 1. A Production Example of Immunochromatographic Test Strip of the Present Invention

### 1) Production of Colloidal Gold-Labeled Anti-CRP Antibody and Colloidal Gold-Labeled Anti-Hb Antibody (Conjugates)

The anti-CRP monoclonal antibody (Clone: FERM BP-11344) and the anti-human Hb monoclonal antibody (Clone: #69202) were prepared in accordance with the following buffer solution conditions and antibody concentrations of i) and ii), and 1 mL of each antibody solution was added to 20 mL of a 1 OD/mL colloidal gold solution (particle diameter: 40 nm) and stirred at room temperature for 10 minutes. After 2 mL of a 10 % bovine serum albumin (BSA) aqueous solution was added to each of the colloidal gold/antibody mixtures and further stirred for 5 minutes, the mixtures were centrifuged at 10 °C at 10,000 rpm for 45 minutes to obtain sediments (conjugates). To the acquired conjugates, 1.2 mL of Conjugate Dilution Buffer (manufactured by Scripps Laboratories) was added to suspend the conjugates. The absorbance of the conjugates was measured at the maximum absorption wavelength.
i) FERM BP-11344 (20 µg/mL), 2 mmol/L phosphate buffer solution (pH 7.0)
ii) #69202 (80 µg/mL), 2 mmol/L borate buffer solution (pH 9.0) (antibodies are described as clone names of hybridomas producing the antibodies for convenience)

### 2) Production of Conjugate Pad

The anti-CRP monoclonal antibody-sensitized conjugate and the anti-Hb monoclonal antibody-sensitized conjugate produced in (1) above were diluted to 20 OD/mL and 10 OD/mL, respectively, with a 20 mmol/L Tris-hydrochloric acid buffer solution (pH 7.5) containing 1.33 % casein and 4 % sucrose to prepare a conjugate solution. A glass fiber pad having a certain volume (No. 8964 manufactured by Pall Corporation) was impregnated with 1.2 volumes of the conjugate solution relative to the volume of the pad. The pad was dried at 70 °C for 30 minutes in a dry oven to obtain a conjugate pad. If an additive such as a sensitizer is added as needed, a necessary amount may be added to the conjugate solution before performing the same operation.

### 3) Production of Anti-CRP Antibody- and Anti-Hb Antibody-Immobilized Membrane

The anti-CRP monoclonal antibody (Clone: FERM BP-11345) and the anti-Hb monoclonal antibody (Clone: #69209) were prepared at 1 mg/mL as a 10 mmol/L phosphate buffer solution (pH 7.2) containing 2.5 % sucrose to apply the anti-CRP monoclonal antibody onto a nitrocellulose membrane (Millipore, HF240 or HF180) at a position inside one end of the short sides and the anti-Hb monoclonal antibody at an interval of about 5 mm by using an immunochromatography dispenser "XYZ3050" (BIO DOT) set to 0.75 µL/cm in a line shape. The membrane was dried at 70 °C for 45 minutes in a dry oven to obtain an antibodies-immobilized membrane.

### 4) Production of Sample Pad

A glass fiber pad (Lydall) cut to a certain volume was impregnated with 1.15 volumes of a 20 mmol/L Tris-hydrochloric acid buffer solution (pH 7.2) containing 24 mmol/L NaCl, 0.5 % sucrose, and 30 mmol/L ethylenediaminetetraacetic acid relative to the volume of the pad. The pad was dried at 70 °C for 45 minutes in a dry oven to obtain a sample pad.

### 5) Production of Test Strip

The antibody-immobilized membrane (b) was affixed to a plastic adhesive sheet (a) and the application portions of the anti-CRP antibody (c) and the anti-Hb antibody (d) were so arranged that the former was located on the upstream side of the development/spread, and the 3rd pad (i) consisting of a grass fiber pad was further mounted. The conjugate pad (e) produced in 2) was then disposed and mounted and the sample pad (f) produced in 4) was disposed and mounted to overlap the conjugate pad while the absorbent pad (g) was disposed and mounted on the end of the other side. Finally, a polyester film (h) was disposed and mounted for lamination on the upper surface to cover the antibody-immobilized membrane and the absorbent pad. The structure formed by overlapping the constituting elements as described above was cut to produce the test strip. The test strip was stored in or mounted on a dedicated plastic housing (having a sample addition window and a detection window not depicted in Fig. 1) at the time of an assay to implement a form of an immunochromatographic test device. Fig. 1 is a schematic of a structure of the test strip.

### 6) Production of Diluting Solution

Preservative was added to 10 mmol/L phosphate buffer solution containing sodium hexylsulfate at a final concentration of 0.1 % and the liquid acquired by filtration through a 0.45 µm filter was used as the diluting solution of the reagent.

### [Example 1] CRP Detection Method Using Sandwich Immunochromatography

### (1) Specimen

From one healthy individual agreed to blood collection, 5 mL of blood was collected by using an EDTA-2K vacuum blood collection tube and plasma was fractionated by centrifugation. After the fractionated plasma was caused to pass through an anti-CRP antibody-column to remove CRP, a recombinant CRP was added to 1 mL of the CRP-removed plasma to prepare CRP-containing plasma corresponding to 3.0 mg/dL, which was defined as Specimen 1. One (1) mL of the CRP-removed plasma without addition of the recombinant CRP was defined as Specimen 2. For the recombinant CRP, rCRP-C-reactive protein (recombinant) (manufactured by Oriental Yeast Co., Ltd.) was used.

### (2) Preparation of Sample

Portions of Specimens 1 and 2 were diluted by a factor of 101 with the diluting solution and used as normally operated samples. The diluting solution itself was used as an improperly operated sample to which specimen is forgotten to be added.

### (3) Testing Method

To the sample pad window of the immunochromatographic test strips, 120 µL of the normally operated samples and the improperly operated sample, respectively, was added to measure the reflected light intensity of the test line (CRP measurement) and the control line (Hb capture) of the detection window of the test strips after five minutes by using the immunochromatography reader ICA-1000 (Hamamatsu Photonics K.K.).

### (4) Result

In the case of the normally operated samples, Specimen 1 resulted in coloring recognized in the test line for measuring CRP concentration and the control line for capturing the Hb-label complex, and Specimen 2 resulted in coloring recognized only in the control line. Therefore, it can be determined that specimen was contained in each of the samples. On the other hand, the improperly operated sample resulted in no coloring recognized in the test line or the control line and the sample was determined as a sample to which specimen is forgotten to be added (Table 1).

**[Table 1]**

| Samples | | Test line (mAbs) | Control line (mAbs) |
|---|---|---|---|
| Normally operated samples | Specimen 1 (with CRP) | **342.5** | **257.0** |
| | Specimen 2 (w/o CRP) | **0** | **234.6** |
| Improperly operated sample (without specimen) | | **0** | **0** |

### REFERENCE SIGNS LIST

(a) plastic adhesive sheet
(b) antibody-immobilized membrane
(c) anti-CRP antibody
(d) anti-hemoglobin antibody
(e) conjugate pad
(f) sample pad
(g) absorbent pad
(h) polyester film
(i) 3rd pad

FERM BP-11344
FERM BP-11345

### [Reference to Deposited Biological Material]

### 1) FERM BP-11344

i) Name and address of depository institution at which the biological materials were deposited
   International Patent Organism Depositary, National Institute of Advanced Industrial Science and Technology
   Tsukuba Central 6, 1-1-1 Higashi, Tsukuba, Ibaraki 305-8566, Japan
ii) Date of biological material deposit in the depository institution of i) November 26,2009
iii) Accession number for the deposition assigned by the depository institution of i)

### FERM BP-11344

### (2) FERM BP-11345

i) Name and address of depository institution at which the biological materials were deposited
   International Patent Organism Depositary, National Institute of Advanced Industrial Science and Technology
   Tsukuba Central 6, 1-1-1 Higashi, Tsukuba, Ibaraki 305-8566, Japan
ii) Date of biological material deposit in the depository institution of i) November 26, 2009
iii) Accession number for the deposition assigned by the depository institution of i)
   FERM BP-11345

## Claims

1. An immunochromatographic detection method of detecting an analyte in a sample acquired by diluting a specimen, comprising the steps of:
1) providing a sample to a sample pad of an immunochromatographic test strip comprising
a) a sample pad,
b) a conjugate pad disposed downstream relative to the sample pad, the conjugate pad comprising
a label to which a first antibody to an analyte is immobilized, and
a label to which a first antibody to a control component is immobilized, the control component being contained in the specimen and different from the analyte, and
c) an insoluble membrane which is disposed downstream relative to the conjugate pad and to which a second antibody to the analyte and a second antibody to the control component are immobilized;
2) detecting on the insoluble membrane the presence or absence of a complex of the analyte and the first and second antibodies to the analyte;
3) detecting on the insoluble membrane the presence or absence of a complex of the control component and the first and second antibodies to the control component; and
4) determining that no specimen is contained in the sample if no complex can be detected at the detecting step of 3),
wherein the specimen is plasma or serum, and
wherein the control component is hemoglobin.

2. The detection method of claim 1, wherein the item (b) of the immunochromatographic test strip further comprises a second control component not contained in the sample, wherein the item (c) of the immunochromatographic test strip further comprises a detection reagent for the second control component, and wherein the method further comprises the step of
5) detecting on the insoluble membrane the presence or absence of the second control component.

## Patentansprüche

1. Immunochromatographisches Erfassungsverfahren zur Erfassung eines Analyten in einer Probe, die durch Verdünnung eines Probekörpers erhalten wurde, umfassend die folgenden Schritte:
1) Bereitstellen einer Probe auf einem Probenpad eines immunochromatographischen Teststreifens, umfassend
a) ein Probenpad,
b) ein Konjugatpad, das relativ zum Probenpad stromabwärts angeordnet ist, wobei das Konjugatpad Folgendes umfasst,
eine Markierung, an welcher ein erster Antikörper gegen einen Analyten immobilisiert wird, und
eine Markierung, an welcher ein erster Antikörper gegen eine Kontrollkomponente immobilisiert wird, wobei die Kontrollkomponente im Probekörper enthalten ist und sich von dem Analyten unterscheidet, und
c) eine unlösliche Membran, die relativ zum Konjugatpad stromabwärts angeordnet ist und an welcher ein zweiter Antikörper gegen den Analyten und ein zweiter Antikörper gegen die Kontrollkomponente immobilisiert ist;
2) Erfassen an der unlöslichen Membran die Anwesenheit oder Abwesenheit eines Komplexes des Analyten und der ersten und zweiten Antikörper gegen den Analyten;
3) Erfassen an der unlöslichen Membran die Anwesenheit oder Abwesenheit eines Komplexes der Kontrollkomponenten und der ersten und zweiten Antikörper gegen der Kontrollkomponente; und
4) Bestimmen, dass kein Probekörper in der Probe enthalten ist, falls im Erfassungsschritt 3) kein Komplex erfasst werden kann,
wobei der Probenkörper Plasma oder Serum ist, und
wobei die Kontrollkomponente Hämoglobin ist.

2. Erfassungsverfahren nach Anspruch 1, wobei Element (b) des immunochromatographischen Teststreifens ferner eine zweite Kontrollkomponente umfasst, die nicht in der Probe enthalten ist, wobei Element (c) des immunochromatographischen Teststreifens ferner ein Erfassungsreagenz für die zweite Kontrollkomponente umfasst und wobei das Verfahren ferner den folgenden Schritt umfasst,
5) Erfassen an der unlöslichen Membran die Anwesenheit oder Abwesenheit der zweiten Kontrollkomponente.

## Revendications

1. Procédé de détection immunochromatographique pour détecter un analyte dans un échantillon obtenu en diluant un spécimen, comprenant les étapes de :
1) fournir un échantillon sur un tampon d'échantillon d'une bande de test immunochromatographique comprenant :
a) un tampon d'échantillon,
b) un tampon conjugué ménagé en aval par rapport au tampon d'échantillon, le tampon conjugué comprenant :
un marquage sur lequel un premier anticorps d'un analyte est immobilisé, et
un marquage sur lequel un premier anticorps d'un composant témoin est immobilisé, le composant témoin étant contenu dans l'échantillon et différent de l'analyte, et
c) une membrane insoluble qui est ménagée en aval par rapport au tampon conjugué et sur laquelle un second anticorps de l'analyte et un second anticorps du composant témoin sont immobilisés;
2) détecter sur la membrane insoluble la présence ou l'absence d'un complexe de l'analyte et des premier et second anticorps de l'analyte ;
3) détecter sur la membrane insoluble la présence ou l'absence d'un complexe du composant témoin et des premier et second anticorps du composant témoin ; et
4) déterminer qu'aucun spécimen n'est contenu dans l'échantillon si aucun complexe ne peut être détecté à l'étape de détection 3),
dans lequel l'échantillon est du plasma ou du sérum, et
dans lequel le composant témoin est l'hémoglobine.

2. Procédé de détection selon la revendication 1,
dans lequel l'élément (b) de la bande de test immunochromatographique comprend en outre un second composant témoin non contenu dans l'échantillon,
dans lequel l'élément (c) de la bande de test immunochromatographique comprend en outre un réactif de détection du second composant témoin, et dans lequel le procédé comprend en outre l'étape de :
5) détecter sur la membrane insoluble la présence ou l'absence du second composant témoin.
